# EUROPEAN PATENT APPLICATION

(11) **EP 4 227 385 A1**
(43) Date of publication of application: **16.08.2023**
(21) Application number: 21876777.0
(22) Date of filing: 23.09.2021
(51) Int. Cl.: C10L 1/02, C10G 3/00, C07C 1/20, C07C 29/34

(54) **METHOD FOR PRODUCING RENEWABLE AVIATION KEROSENE**

(30) Priority: 09.10.2020 BR 102020020883
(71) Applicant: Petroleo Brasileiro S.A. - PETROBRAS, 20031912 Rio de Janeiro (BR)
(72) Inventor: MENEGASSI DE ALMEIDA, Rafael, 22775-033 Rio de Janeiro (BR)
(74) Representative: Clarke, Modet y Cía., S.L.
(86) International application number: PCT/BR2021/050406
(87) International publication number: WO 2022/073087

(57) **Abstract**

The present invention relates to a renewable aviation kerosene (bioQAV) production process, the process of which involves the conversion of ethanol and optionally methanol, CO₂, syngas or mixtures thereof, from renewable sources, in a hydrocarbon stream in the aviation kerosene range. The stream has mainly alkyl aromatics, and can be used as partial component of aviation kerosene, fully or partially hydrogenated to alkyl naphthenes.

## Description

### Field of the Invention

The present invention relates to a production process of renewable aviation kerosene (bioQAV) from ethanol from fermentation of sugars applied in the area of biofuels and renewable energy sources, aiming at converting ethanol into at least 50% of C8+.

### Description of the State of the Art

Currently, there is a need in the art of obtaining biofuels, especially renewable aviation kerosene (bioQAV) to replace fossil fuels.

The main route of obtaining bioQAV considered in the art is the hydroprocessing of vegetable oil (Hydroprocessing of Vegetable Oils, HVO), generating paraffins that are subsequently hydroisomerized for use as aviation kerosene (QAV). However, there is already production of biodiesel using the same raw material, which limits its availability. Further, the chain size of paraffins in triglycerides limits the amount of HVO which can be used as bioQAV. To make matters worse, oil productivity plant per unit of planted hectare, for crops such as soy, is low, usually less than 500 L/ha. Other crops such as sugar cane typically allow 65 to 95 ton/ha per year, resulting in ethanol of up to 8.550 L/ha, with potential to increase. That is, it can be produced almost 20 times more ethanol than soybean oil in the same area, which increases with the possibility of cellulosic ethanol and energy cane.

Ethanol from a renewable source is usually produced by fermentation of sucrose by yeasts, such as *Saccharomices Cereviseae.* In addition to sucrose, other sugars such as glucose obtained from starch and xylose obtained from hemicellulose can be fermented. Sucrose can be obtained of sugar cane. From sugarcane bagasse, cellulose can still be converted to glucose and hemicellulose to xylose and other sugars, also fermentable. Assuming the fermentation of the glucose molecule, with 6 carbons, 4 carbons will result in 2 ethanol molecules and 2 carbons in 2 molecules of CO₂. In this way, each molecule of ethanol obtained also generates an unused CO₂ molecule. After fermented production with 7 to 10% ethanol in aqueous medium, there is still great energy demand in the distillation step. The energy for distillation is generated by burning of a significant part of the bagasse. Having a process that takes advantage of the ethanol without the need for water separation will provide more raw material for ethanol production. Further, it is interest in the art of processes that can additionally take advantage of the large amount of CO₂ generated in fermentation.

Several routes allow the conversion of ethanol to middle distillates, and QAV in particular. The routes usually involve the reactions of dehydration of ethanol to ethene, followed by oligomerization of olefins.

The art teaches the dehydration of ethanol to ethene as a first step of conversion processes, according to the reaction: Ethanol → Ethene + H₂O.

Ethene can then be oligomerized to components with size of a molecule compatible with diesel and QAV.

More recently, the alcohol coupling Guerbet reaction is considered, according to EAGAN, N. M. et al. "Chemistries and processes for the conversion of ethanol into middle-distillate fuels", Nature Reviews Chemistry, v.3, p.223 to 249, 2019.

The Guerbet reaction initially involves the reaction: 2 Ethanol → 1-butanol + H₂O and can occur successively.

The patent US 4134926 teaches the dehydration of ethanol in acid catalysts such as alumina, silica-aluminas, activated clays and zeolites, at least 370 °C, and in a fluidized bed to facilitate its regeneration. Lower temperatures favor the formation of diethyl ether, not the dehydration to ethene.

The patent US 4847223 teaches the dehydration of ethanol in liquid phase, without water separation, in superacid catalyst combining zeolite or silica-aluminas and trifluoromethanesulfonic acid (CF₃SO₃H), at temperatures from 170 °C to 225 °C. The document highlighted the importance of processing ethanol dilute aqueous solution, thus produced in the fermentation of sugars.

In possession of olefins such as ethylene, it is possible to perform the oligomerization to olefins of higher molecular weight, as taught by the reference US 8802905.

One class of such ethylene oligomerization processes with high conversion is by transition metals, usually homogeneous, as the Ziegler-Natta process, taught in the patent US 2943125. However, the nature of this oligomerization is of little selectivity, with a product distribution of Schulz-Flory type. The same distribution is achieved in supported catalysts such as Ni, for ethylene oligomerization. This makes more than one step of oligomerization necessary, such as production of butylenes or greater and subsequent oligomerization step by acid catalysis, as taught by US 8957270 and US 9771533. The patent US 9840676 teaches the trimerization of ethylene to hexene and its further dimerization and trimerization, with final hydrogenation, to C12 and C18 paraffins.

The oligomerization of ethylene in one step, with selectivity, is difficult by classical heterogeneous acid catalysis, being preferable the oligomerization of butylenes or greater, including recycling of products below of the distillate range, as taught by documents US 7271304, US 9644159 and US 9663415. Even the conversion of ethanol and/or ethylene to C3 to C6 olefins for further oligomerization.

The patent US 8378160 teaches how to obtain isobutanol by fermentation, dehydration to olefins, oligomerization in acid catalyst and subsequent hydrogenation. It also teaches the transformation of isobutenes into aromatics, and the alkylation of olefins with the aromatics obtained.

Due to the difficulties in obtaining ethanol, removal of water, transformation, separation, purification and recycling of olefins, the art sought processes for the direct conversion of ethanol to hydrocarbons in the range of distillates. US 9475999 teaches such a process, in which a first reaction in ethanol zeolitic acid catalyst to mostly olefins of 2 to 5 carbons is carried out, and subsequent conversion into greater severity generating olefins, aromatics, paraffins and naphthenics, of which about 30% has a boiling point in the distillates range. The patent US 4925996 also teaches a two-step conversion process. These processes are similar to those developed with zeolitic catalysis in the 1980s, where methanol obtained by synthesis gas was converted into gasoline or diesel (Methanol to Diesel, MTD).

As the initial oligomerization step may present low selectivity and problems such as catalyst deactivation, an attempt was made to alternatives for the production of longer chain components from ethanol, like the Guerbet reaction.

The Guerbet reaction is a coupling reaction of alcohols. In the case of ethanol, it involves dehydrogenation to acetaldehyde, aldol self-condensation of acetaldehyde and hydrogenation of the intermediate to butanol. The reaction can occur sequentially, generating C4-C6 alcohols, C6-C8 alcohols or even C12-C16 alcohols, and the process can be combined with another acid oligomerization after dehydration of olefins of different chain sizes.

The art teaches the use of up to 30% Guerbet alcohols derived from ethanol as direct components of bioQAV, according to the document US 9528058, although there are disadvantages to using oxygenates such as fuel stability and the lowest calorific value.

Due to the difficulties involved in converting ethanol, other types of fermentation were sought, obtaining products of greater chain size and facilitated oligomerization. An example is the production of isobutanol by fermentation, more easily converted to isobutene and oligomers, as taught by US 8975461.

As taught by US 8907150, some microorganisms in fermentation produce acetone, which is easily converted into mesitylene acid (1,3,5-trimethylbenzene) catalyst and other aromatic and phenolic alkyls. Mesitylene can be used in up to 30% in QAV from Fischer-Tropsch to improve some of its properties.

Acetobutyl fermentation produces a mixture of butanol, acetone and ethanol. US 9790444 patent teaches the production of higher molecular weight branched paraffin hydrocarbons from the reaction of condensation of alcohols and aldol in Guerbet type catalysts, with basic function in support, such as hydrotalcite, and hydrogenating/ dehydrogenating function such as Pd and Cu. The document teaches coupling of acetone with one or more primary alcohols, resulting in higher molecular weight branched ketones. The patent, however, requires that the water content in the reaction load is less than 5% due to its inhibition effect.

The document US 10351487 teaches the direct processing of aqueous ethanol solution in mixed oxide containing at least Zn and Zr, to functionalized light compounds, mainly isobutylene. Isobutylene, ethylene, propylene and acetone (in addition to water) are formed as main products, but unfortunately CO₂ in the order of 20 to 25% of the total carbon. At same patent they teach different formulations of the catalyst and conditions which can increase the acetone yield. As a by-product of the synthesis of isobutylene and acetone show phenols with up to 8 carbons.

The conversion of ethanol to acetone is known in the art, as taught by US 1663350. Ethanol and water are processed at temperatures from 250 °C to 650 °C, with metal oxide from groups 5 to 10 of the periodic table, preferably supported, most preferably with alkaline earth metal oxide. Examples are Fe₂O₃ supported on ZnO-CaO. The stoichiometry of the reaction is: 2 Ethanol + H₂O → CH₃COCH₃ + CO₂ + 4H₂. To each 2 molecules of ethanol 1 molecule of acetone is formed and one carbon is lost as CO₂.

To avoid obtaining intermediates such as acetone or olefins of greater number of carbon, the several steps of transformation and processing, the art also teaches the direct fermentation of sucrose to product as farnesene and its hydrogenation to farnesane, as US 7399323.

However, the ethanol route has a higher fermentation yield of sugars, even if to each 6 carbons in a unit of sugar (glucose), 2 carbons are lost as CO₂.

In this sense, it would be interesting to directly convert the sugars to hydrocarbons, as taught in US 20160326448. Another conversion route of sugars involves the hydrogenation of sucrose or glucose to polyols, the reforming the aqueous phase of polyols to intermediate oxygenates (Pt/Pd in zirconia) and finally the production of alkyl-aromatics in acid catalysis with nickel supported on ZSM-5, the product being SAK (Synthetic Aromatic Kerosene) used as a component of QAV. However, the use of SAK in bioQAV is limited to typically 20 to 25% for aromatics effects in quality.

In addition to the direct use of sugars and other components of biomass in direct processes for conversion to fuels, would also be interesting the use of the product CO₂ from the fermentation itself as well as the potentially generated in bagasse burning.

Another possible route is the generation of methanol from gasification biomass, and conversion of this methanol to bioQAV using MTD processes (Methanol to Diesel), but several processing, separation and treatment are necessary. Further, in the case of sugarcane, significant amount of bagasse is burned to provide energy for the distillation of the diluted ethanol from fermentation, decreasing its availability, and the remainder is burned to produce electricity.

Despite the current and future use of bioQAV, there is no defined, advantageous, production process of the same, either by low agricultural productivity, several processing steps, low yield, non-use of carbon available as CO₂. The absence of a more favorable obtaining process is evidenced by the multitude of inventions in the art.

In the study by WEI, H. et al. "Renewable bio-jet fuel production for aviation: A review", Fuel, v. 254, 115599, 2019, they teach an overview of the conversion technologies, economic evaluation, environmental influence and development status of aviation biofuels. In addition, it teaches methods for obtaining biofuels for aviation, but it does not specify their compositions and concentrations used in each step of each method.

Thus, no prior art document discloses a process for the production of renewable aviation kerosene from ethanol such as that of the present invention.

In order to solve such problems, the present invention was developed, through which the production of bioQAV through conversion of ethanol and additionally methanol and/or CO₂ or synthesis gas into alkylaromatics by basic catalysis route plus hydrogenating/dehydrogenating function.

### Brief Description of the Invention

The present invention relates to a production process of renewable aviation kerosene (bioQAV), characterized in that the reaction of ethanol and optionally methanol and/or CO₂ and/or synthesis gas and mixtures thereof, in the presence of hydrogen, into a catalyst combining basic and hydrogenating/dehydrogenating function, preferably heterogeneous, resulting in alkyl aromatics.

The catalyst and conditions of the invention allow the alcohol combination Guerbet reaction, with generation of acetone and coupling of the same and other intermediates to alkyl-aromatics in the QAV distillation range. The alkyl aromatics preferentially undergo hydrodeoxygenation to conversion of oxygenates and remaining olefinic unsaturation and optionally hydrogenation of alkyl aromatics to alkyl naphthenics.

In the preferred embodiment of the present invention, hydrogen generated in the first step of the alcohol coupling reaction is used to hydrogenation of at least part of the alkyl aromatic to alkyl naphthenic.

In another embodiment of the present invention methanol or precursors of the same can also be combined with ethanol in the coupling reaction. An advantageous case is the use of CO₂ itself generated in the fermentation of sugars to bioethanol.

### Brief Description of the Drawings

The present invention will be described in more detail below, with reference to the attached figure which, in a schematic and non-limiting way of the inventive scope, represents an example of its realization. In the drawing, there is:

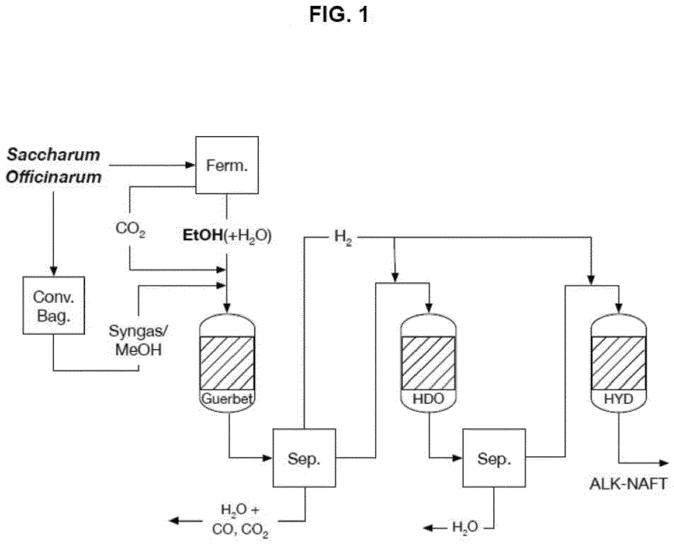

- Figure 1 illustrating the process scheme of the present invention from ethanol from fermentation of sugar cane sugar (*Saccharum Officinarum*)*.* The Figure shows fermentation steps of sucrose (Ferm.), bagasse conversion (Conv. Bag.) to synthesis gas (Syngas) and/or methanol (MeOH), the coupling step (Guerbet), followed by separation (Sep.), where organic liquid effluent plus H₂ generated go to the hydrodeoxygenation reactor (HDO), and finally hydrogenated to alkyl naphthenes (ALK-NAFT) in a final hydrogenation reactor (HYD).

### Detailed Description of the Invention

The production process of renewable aviation kerosene (bioQAV) from ethanol from the fermentation of sugars, according to the present invention and illustrated, in a particular embodiment of the invention, in Figure 1, is characterized by the coupling reaction of ethanol, being catalyzed by a combination of hydrogenating/dehydrogenating function and basic function, as catalysts for the Guerbet reaction. The catalyst of present invention, in addition to the Guerbet alcohol coupling reaction, also promotes cyclization to aromatics. Without limiting the present invention to understanding the reaction mechanism, it is believed to occur parallel to the Guerbet reaction the synthesis reaction of acetone or intermediate and condensation thereof and the same between the alcohols by aldol condensation.

The Guerbet reaction involves the reaction of a primary and/or secondary alcohol (or alcohol mixture), occurring in the following steps:
- dehydrogenation of alcohols (primary) to aldehydes: 2 molecules of ethanol dehydrogenates acetaldehyde (generating 2 H₂); other primary and secondary alcohols also dehydrogenate (methanol to formaldehyde, butanol to butanal);
- aldol condensation: the aldol condensation between two molecules containing carbonyls (aldehyde or ketone), followed by elimination of water; generating the intermediate enone (ab-unsaturated acetone) (instead of water elimination the intermediate molecule of the coupling of two ethanol molecules can decarboxylate generating acetone);
- hydrogenation: the enone is hydrogenated, with the H₂ generated from the original hydroxyls of alcohols, resulting in butanol;
- synthesis of acetone, or intermediate: occurs from the intermediate of the Guerbet reaction decarboxylating before eliminating water, resulting in:

   2 CH₃CH₂OH + H₂O → CH₃COCH₃ + CO₂ + 4 H₂

   The acetone synthesis reaction depends on water as a reagent, which comes from the Guerbet condensation reaction and through the formation of ethers (e.g. ethyl ether) or dehydration plus dehydrogenation of cyclic compounds. In addition to the water generated in the reactions, more water can be powered to the load. More preferably, the solution of ethanol from fermentation.

In the reaction steps described, in addition to ethanol, corresponding intermediate aldehydes and ketones are employed, or even acetone, in addition to other alcohols such as formaldehyde, from methanol. In addition, methanol precursors can be used to formation of methanol *in situ*, or mixtures of methanol and precursors.

Acetone is very reactive, and by basic catalysis it reacts to oxide mesityl, mesitylene and isophorone, and in its turn to phenolics and aromatics.

The catalyst and conditions of the present invention allow both routes happen together, and that aldehydes from both the ethanol and derivatives and acetone and derivatives react with each other, for aldol condensation. Furthermore, the catalyst can carry out the reaction of coupling of intermediate phenolic compounds with alcohols, including methanol.

Additionally, the catalyst of the present invention catalyzes the CO₂ hydrogenation reactions and the water-gas-shift reaction and its reverse during the reaction, resulting in CO₂ formed in the reaction or fed to the reactor can be converted into methanol and formaldehyde, continuing the reactions of aldol condensation.

In a preferred embodiment of the present invention the CO₂ generated in fermentation for ethanol production is fed to the coupling reactor.

In another preferred embodiment of the present invention, the bagasse of sugar cane or biomass is sent to gasification to generate methanol or sent directly to the ethanol coupling reaction.

Although the invention used ethanol from a renewable energy source and preferably methanol from a renewable source, to obtain renewable aviation kerosene, totally or partly, ethanol and methanol (or methanol precursors such as CO₂) from other, non-renewable sources, can be used, and the renewable content of the aviation kerosene so-called renewable is proportional to the renewable content of reagents.

In case of using methanol, preferably the coupling reaction is carried out in two steps, to avoid unwanted formation of C3 oxygenates which are more time consuming to react. So, methanol and formaldehyde are more rapidly reacted with higher molecular weight alcohols and phenols, increasing the content of products in the middle distillate range.

Other parallel reactions are known, but occur to a lesser extent grade, generating dienes and olefins. These lightweight compounds can be recycled to the reaction or used for aromatic alkylation.

The mixture of alkyl aromatics and oxygenated products from condensation reactions can be hydrodeoxygenated in a second step of oxygen removal reaction, particularly hydrodeoxygenation (HDO). Preferably, the product of the ethanol coupling reaction passes through HDO, both to provide stability to the use of the stream as bioQAV and to facilitate the hydrogenation of alkyl aromatic to alkyl naphthenics in a final hydrogenation step.

Furthermore, it was discovered that a significant amount of hydrogen is generated in coupling reactions, including by dehydrogenation in formation of aromatics, and the H₂ generated can contribute to the conversion of CO₂ and CO and subsequent hydrodeoxygenation and hydrogenation reactions, decreasing the demand for external H₂.

Alternatively, intermediate oxygen compounds can be sent back to the first step of the ethanol coupling reaction.

Oxygen removal may occur for at least two mechanisms. In addition to direct HDO, it can be catalyzed by a combination of hydrogenating and acidic function, in which the carbonyls of the aldehydes are hydrogenated to alcohols, dehydrated alcohols to olefins and resulting olefins hydrogenated or not to isoparaffins, depending on the catalyst and hydrodeoxygenation conditions.

In addition to the aforementioned mechanism of dehydration and hydrogenation, direct HDO from alcohols and carbonyls may occur.

The alkyl aromatics products of the alcohol coupling steps and HDO can be further fully or partially hydrogenated to alkyl naphthenes. The reaction is advantageous to gain density, use H₂ generated in the reaction itself, and finally enable greater use of the stream as bioQAV, which has a maximum limit of aromatics in the specification.

The step of HDO and hydrogenation of aromatics can also take place in the same reactor or same catalyst.

Usually, the HDO step is separated from the hydrogenation step of aromatics, since some typical HDO catalysts (e.g., Mo or W sulfides, promoted or not by Co and Ni) has little activity for hydrogenation of aromatics.

Both the first reaction step of coupling alcohols and the subsequent HDO step can take place in the same reactor or in different reactors. Preferably, in different reactors, with recovery of unreacted oxygenates in the aldol coupling reaction step and their recycle to the coupling reactor.

In the ethanol coupling reaction step, MeOH can be additionally used as a filler, or the same can be generated *in situ* from CO and hydrogen, CO₂ and hydrogen or a mixture thereof. Actually, CO₂ results from the parallel/intermediate acetone synthesis reaction itself. The mixtures of CO, CO₂ and H₂ gases can still be combined with MeOH in the feed of the reactor. In addition to methanol, formaldehyde can also be fed to the reactor, since it is the intermediate of the reaction produced by methanol dehydrogenation. However, the use of MeOH and/or combination of the same with synthesis gas are preferred, since formaldehyde can polymerize, obstructing access to catalyst sites. Under the conditions of present invention, the calculation of the chemical equilibrium indicates that the amount of formaldehyde formed is small, and the absence of formaldehyde in the analysis of the product indicates that it reacts quickly.

The hydrogenolysis reaction of methanol to CO and H₂ is endothermic, while the synthesis reaction of MeOH from CO and H₂, exothermic. It can be advantageous the combination of methanol with synthesis gas (CO and H₂) in the load, in order to reduce the need for heat supply in the reaction conditions, making the temperature profile in the reactor close to the isothermal, favoring the coupling reaction.

Thus, the coupling reaction with ethanol can be either powered by mixtures of CO and H₂ or CO₂ and H₂ or mixture thereof or be fed by MeOH or mixture of gases. The presence of H₂ has an effect in the methanol synthesis reaction when the feed contains CO (synthesis gas). If CO₂ is used as a reagent, higher levels of H₂ are required for hydrogenation. For CO to be hydrogenated to methanol, 2 molecules of H₂, for CO₂ to methanol 3 molecules of H₂ are required. A coupling reaction occurs with compounds containing 1 carbon, either MeOH or mixtures of H₂ and CO or CO₂ or even formaldehyde, provided that sufficient conditions exist for the formation of methanol and for formaldehyde in its turn.

The MeOH synthesis reaction can be performed in different reactors prior to mixing with ethanol or be formed *in situ* in parallel to the coupling reaction.

Catalysts for the coupling reaction step have a basic function and hydrogenating/dehydrogenating function. Typical temperatures are greater than 150 °C, preferably greater than 250 °C, more preferably greater than 350 °C. Temperatures greater than 450 °C are unnecessary, and lead to increased formation of secondary reactions, although temperatures up to 550 °C can be used.

Higher pressures thermodynamically favor the alcohol coupling reaction. Typical pressures are at least 1 bar, usually greater than 5 bar, preferably greater than 10 bar, more preferably greater than 20 bar and less than 60 bar, more preferably less than 100 bar. Pressures greater than 60 bar leads to smaller gains, not being the preferred conditions, although pressures of up to 200 bar can be used. Condition between 20 and 40 bar is preferred and sufficient for the reactions. In case of feeding higher amounts of CO₂ in the reaction, higher pressures may be required, but limited to the values previously described.

As H₂ is generated in the reaction, it is interesting to have pressure high enough to use the same gas (after separation of CO₂ and CO) for the HDO step. Preferably the gaseous effluent from the coupling reaction is partially sent back to the coupling reaction itself for the synthesis of formaldehyde and its coupling reaction.

Temperature and pressure are correlated, since higher pressures disfavor dehydrogenation and higher temperatures are needed to maintain the same level of dehydrogenation. Pressures very low, however, result in the need for increased reaction time. The conditions of the invention are ideal for the alcohol coupling reaction and the alkyl aromatic synthesis.

Several forms of contact between the catalyst and the reactants are possible in the present invention. Batch mixing reactors, continuous mixing reactors (CSTR), batch or continuous reactors with homogeneous catalysts, reactors with heterogeneous catalysts, fluidized bed or transported (riser) can be used, preferably, continuous reactors with fixed-bed heterogeneous catalysts. The use of heterogeneous catalysts facilitates the separation between the products and the catalyst. Since the ratios between basic and hydrogenating functions are such that catalyst deactivation is small, the preferred form of contact between the reactants and the catalyst is to make the reaction continuous, with the fixed bed catalyst, plug flow. Other continuous reactor schemes are possible, such as mud bed, fluidized bed, but without advantages over the preferred method of fixed-bed continuous reaction. Also, homogeneous catalysts (basic catalyst) and heterogeneous (hydrogenation/ dehydrogenation catalyst) can be combined.

For the continuous reaction in a fixed bed, batch volumes processed per unit reactor volume per unit time (LHSV) are typically 0.1 to 10 h⁻¹, preferably the LHSV (load volume per hour per reactor volume) are from 0.2 to 5 h⁻¹, more preferably from 0.5 to 2 h⁻¹.

The addition of water facilitates the synthesis of acetone, accelerating the coupling reactions, as well as inhibiting the unwanted reactions of etherification.

Further, the ethanol-water solution from the fermentation itself can be used, preferably provided with a previous evaporation of the mixture for purification. The use of ethanol-water solution avoids the high energy demand from distillation to recover ethanol from fermentation. The water of the load plus the one coming from the alcohol coupling reaction and HDO are easily separable from coupling products or HDO, by polarity difference, when an organic and an aqueous phase is formed.

Hydrogen is not necessary for the reaction, since it is generated and consumed in the coupling reaction itself and in the acetone synthesis. The presence of hydrogen, however, may be desired for increase in campaign time and favor alcohols, more easily hydrodeoxygenated than aldehydes or ketones.

It is understood that the operation mechanism of hydrogen is that the presence of hydrogen keeps the catalyst partially reduced, or with the desired degree of reduction of metals hydrogenating/dehydrogenating function, favors the evaporation and hydrogenation of reactive intermediates, such as enone, which could lead to the formation of coke on the catalyst.

Not counting the H₂ required for the synthesis of MeOH when uses mixtures of CO and/or CO₂ in the Guerbet reactor, when H₂ is additionally used, the typical H₂ ratio is between 0 and 500 NL of H₂/L of ethanol, preferably between 0 and 100 NL of H₂/L, more preferably from 20 to 50 NL of H₂/L of ethanol, where NL refers to normal liter, liter of gas in normal conditions of temperature and pressure. Preferably, H₂ is not necessary, but can be used to heat the reactor bed and as catalyst activation or regeneration.

The Guerbet reaction produces H₂O as a by-product, in which it can react with methanol and CO, in the water-gas-shift reaction, resulting in more H₂. Metals with hydrogenating/dehydrogenating capacity are known to catalyze these reactions. As is known in the state of the art, several reactions can occur from MeOH, the main ones mentioned being MeOH to DME, MeOH to formaldehyde, which can in its turn react to methyl formates which break down CO₂ and methane or CO and H₂.

Copper is known to be a catalyst for the hydrogenation of CO and CO₂ to methanol, mainly if promoted by ZnO. Other active metals for reactions are Fe, Ni, Pd, Pt, Rh, Ru, among others.

As part of ethanol and methanol are converted into synthesis gas (CO and H₂) and CO₂ during the reaction under the conditions of the invention, preferably the gaseous phase is fed back to the reactor, using a recycle gas compressor. Part of the gas can be purged and H₂ fed to keep the ratio between H₂ and CO at 2:1 mol/mol. More preferably there is a recycle compressor for recompressing the product gas and sending it back to the reaction.

The means of gas recompression, separation of inert and other by-products such as methane, are known to those skilled in the art, and do not constitute an inventive fact.

Homogeneous alcohol coupling catalysts known are: salts, hydroxides and alkoxides, associated or not with hydrogenating/dehydrogenating function catalysis, homogeneous or not. Preferably heterogeneous catalysts are used, most preferably a metal with hydrogenating/ dehydrogenating function supported on a basic support, or support containing basic component.

As basic supports or components oxides, hydroxides, phosphates, carbonates, carbides of elements of group IA and IIA, alkali and/or alkaline earth metals such as K, Na, Ca, Cs, Sr, Ba and Rb can be used. Only one basic component or mixture of components such as mixed oxides can be used.

Alternatively, supports such as alumina, silicas, zeolites, doped with basic components such as K, Ca or Mg, usually as oxides (K₂O, CaO) can be used.

The supports themselves can be basic, such as alkaline earths metal oxides such as MgO, CaO, SrO, BaO and rare earth oxides such as CeO, La₂O₃, Sm₂O₃. Other oxides provided to the present invention such as support, in addition to Al₂O₃, are ZrO₂, Y₂O₃, ZnO, TiO₂, MoOs and ThO₂. They can be also double component oxides such as ZnO-Al₂O₃, MgO-TiO₂. In general alkali metal ions supported on alumina, silica and the aforementioned oxides, mainly alkaline earth metal oxides. Ions are employed mainly as oxides, but can be non-oxides, such as KF and KCl supported on alumina, and lanthanide imides and nitrides.

Basic zeolites can also be used as basic components in the present invention, which may have alkaline ions by ion exchange, such as Na-X, Cs-X, or have alkali added, such as Cs₂O/Cs-X. Zeolites exchanged with basic metals, already mentioned, transition metals, rare earths, higher valence oxides.

Basic clays, mineral clays, such as limestone, dolomite, magnesite, chrysolite, sepiolite, olivine and hydrotalcite are also known.

Hydrotalcites are lamellar double hydroxides, where a divalent cation (such as Mg, Mn, Fe, Co, Ni, Cu, Zn, Ga) is combined with a trivalent metal (Al, Cr, Mn, Fe, Co, Ni, La), plus a compensating anion between the lamellae. Hydrotalcite can also be doped with additional metal. Hydrotalcites can be calcined or not, resulting in mixed oxides and spinels, depending on the condition.

Hydroxyapatites are also known in the art as basic catalysts.

Perovskites, beta-aluminas, hydroxides and metallic carbonates in general can be also used. Other supports are nitrides, nitrates, metallic or supported sulfides, sulfates, carbides, phosphates and fluorides; activated and impregnated carbons; anion exchange resins; organic bases supported on microporous or mesoporous metallic oxides; in general solid or supported alkalis and alkaline earth metals or basic organometallics. The invention, however, is not limited to the nature of the component which confers the basic function employed in the coupling catalyst, different basic compounds may fulfill the same basic function in reaction.

A support widely used in industry is alumina, mainly gamma-alumina. Gamma-alumina has intrinsic acidity unwanted, but good surface area and pore distribution, and can be doped with alkaline and/or alkaline earth metals such as K, Na, Ca, Cs or Rb.

The hydrogenating/dehydrogenating function in the coupling catalyst can be transition metals, specifically from group VB, VIB, VIIIB, IB, as V, Cr, Mo, W, Fe, Ru, Rh, Re, Co, Ni, Cu, Ag, Sn, Pb, Zn, Mn, Pt and Pd, alone or in combination. Preferably the metals are Cu and/or Ni. Metals may or may not be promoted by other metals such as Zn. Metals can be present as oxides, hydroxides, salts or reduced. Metals may further be present as a homogeneous catalytic system, this supported or not.

It is also possible to use a mixture of distinct catalysts, a basic solid and a hydrogenating catalyst, but preferably the functions, basic and hydrogenating, are combined in the same catalyst. Further, the basic function may be present as homogeneous or heterogeneous catalyst, and likewise the hydrogenating/ dehydrogenating function may be present in homogeneous or heterogeneous form, being possible homogeneous basic phase with supported hydrogenating catalyst or heterogeneous basic phase and homogeneous hydrogenating function. The preferred catalyst, however, combines the two functions in the same heterogeneous catalyst.

In a particular embodiment of the invention, the catalyst is Cu deposited on potassium-doped gamma-alumina. A catalyst prepared containing copper species Cu(I) has more activity for the coupling reactions than fully reduced copper. Cu(I) can be prepared with precursors such as CuCl₂, which reduces to CuCl when reacted with KOH on the surface of the catalyst. A preferred method of preparing the catalyst is deposit CuCl₂ on alumina, calcine and then deposit KOH and then final calcination.

Another promoter of Cu(I) and Cu(II) is the presence of ZnO on the support. Also, ZnO can be combined with K or another basic component. One known support is the mixture of ZnO and Al₂O₃. The use of Cu catalysts in ZnO and Al₂O₃ mixtures is known in industry, especially for the hydrogenation of CO (and CO₂) to methanol.

The contents of Cu in the catalyst may be at least 1% by weight, preferably greater than 2% by weight and less than 10% by weight and preferably in the range of 5% by weight. The KOH content in the alumina is at least 5% by weight and less than 30% by weight, preferably greater than 10% by weight and preferably equal to 20% by weight. Amounts greater than 30% by weight lead to pore occlusion and exacerbated reduction of the catalyst area.

In addition to the preferential use of Cu, another metal can be used together with Cu, as a promoter of dehydrogenation and promoter of methanol activation reactions such as, but not limited to Pd, Pt, Fe.

Unreacted alcohols and aldehydes can be separated from the products from the ethanol reaction and fed back to the coupling reaction, as well as ethers. The entire effluent can still be partially hydrodeoxygenated before of separation.

In addition to alkyl aromatics and other main phenolic compounds other by-products can be formed in the reaction, such as ethyl ether (diethyl ether, DEE), methyl ether (dimethyl ether, DME), ethoxyethane, esters and others oxygenated.

Part of these by-products can be reused through hydrolysis reaction. Hydrolysis is easily carried out by reacting the ether with water on an acid catalyst, such as an acidic ion exchange resin or acid catalysts known in the state of the art. For hydrolysis it is necessary to supply water to the hydrolysis reactor, at least in the stoichiometric ratio.

Alternatively, the direct recycling of the ethers or the mixture of remaining ethers plus alcohols to the main reactor to hydrolysis can be carried out. In this particular case, it is interesting to feed H₂O to the reaction load. More preferably, water can be fed to the reactor coupling load also to disfavor the etherification and dehydration reactions.

Depending on the amount of water present in the product, the effluent of the coupling step separates into two phases. In case of presence of methanol in the product, part of which remains in the aqueous phase, facilitating its separation and reuse in the coupling reaction. Further, liquid-liquid extraction scheme, preferably with water countercurrent to organic phase, can be used to separate methanol and non-ethanol converted. Preferably the ethanol and methanol are separated to be reused in the coupling reaction.

Separation means such as distillation are known in the state of art and do not constitute inventive novelty.

After the alcohol coupling reaction, aldehydes and ketones unreacted are preferentially returned to the reaction. Furthermore, olefins and light dienes also present in the products can be reacted by alkylation with benzene, toluene and styrene to produce higher molecular weight compounds, in the range of C8+.

After the coupling step, follows the HDO and/or hydrogenation step. Without methanol removal after coupling, in HDO this could be converted either to dimethyl ether (by acid function in the catalyst) as hydrogenated to methane, both reactions also generating water. The same can occur with unreacted ethanol, converted to ethane in HDO, which leads to unnecessary consumption of H₂. Likewise, gaseous effluents of CO and CO₂ from the first reaction can also be converted to methane in the HDO step, which is undesirable because it consumes H₂ and does not allow that the carbon is reused in the coupling.

Preferably, for the H₂ to be fed to the HDO and hydrogenation steps CO and CO₂ have been removed. Means for removal of CO₂ and CO are known in the state of the art.

Thus, preferably, the gas containing H₂, CO and CO₂ effluent from the coupling reaction is sent back to the coupling reaction, H₂ being completely or partially removed in order to maintain a molar relationship desired of MeOH and MeOH precursors. Further, it may be necessary to purge of part of the gas to avoid accumulation of inert (light hydrocarbons, mainly methane) formed in the reaction. The methane and light formed in the reaction can be sent to a synthesis gas/MeOH production unit.

Thus, although it is possible to use two beds of different catalysts, one for coupling and another for HDO in the same reactor, it is preferable to separate lighter plus product gas from the coupling step prior to the HDO step to prevent H₂ from being consumed by reaction with CO₂.

Typically, the conversion of ethanol is greater than 75%, more usually greater than 95% under the reaction conditions of the invention, which remainder of ethanol can be separated by means known in the state of the art, as distillation, and sent back to the coupling reactor.

For the subsequent hydrodeoxygenation reaction (HDO), several heterogeneous catalysts are known and widely used in the state of the art.

Commercially available catalysts as hydrotreating catalysts are commonly employed, Mo or W sulfides, promoted by Ni or Co, supported on solids such as alumina, silicas, silica-aluminas, zeolites, hydrotalcites, mixed oxides, spinels, MgO, TiO₂, ZnO, CeO₂, phosphates, sulfonic resins, ZrO₂, sulfated Zr, carbon, active carbon, among others.

Ni-promoted Mo sulfides supported on gamma-alumina hydrotreating catalysts (HDT) are more common. Typical contents are 10 to 20 wt%, typically 15 wt% Mo plus 5% Ni (such as MoOs and NiO) supported on Al₂O₃. Optionally, W sulfides, or mixtures thereof with Mo, can be used, and alternatively Co in place or in addition to Ni as a promoter. These catalysts are widely commercially available, being used in the HDT of petroleum fractions. It is necessary to perform sulfidation prior to using the catalyst, or use presulfided catalyst, also commercially available. When using sulphide catalysts, it may be necessary to dop the load with sulfur compounds continuously or intermittently to maintain the sulphide catalyst.

In addition to sulphide catalysts, metals can be used fully or partially reduced as Pt, Pd, Ru, Ni, Cu, Mo, W, Co, Ir, Rh, Au, Ce, Fe, Mn, Ga, Pb, Bi. Metals are usually supported on the same supports described above. In addition to reduced metals, other catalysts such as oxides, phosphates, carbides and nitrides, such as MoOs, NiP, MoC₂ and CoNₓ are known.

Specifically, MoOs, with vacancy due to the presence of H₂, has the ability to remove carbonyl oxygen and alcohols resulting in alpha-olefin. In addition to MoO₃, Mo carbides and nitrides can make direct HDO from alcohols and carbonyls. The working mechanism of catalysts is by oxygen vacancy created by H₂, in a mechanism Mars-van Krevelen reverse C-O bond activation. In addition to MoO₃ the same mechanism also occurs with RuO₂, IrO₂, PdO and Rh₂O₃. Other catalysts with lower activity are SnO₂, ZnO, VO₂, TiO₂ and CeO₂, in addition to CuO, Ag₂O and Au₂O₃.

Some supports are oxophilic, have an affinity for oxygen, which may favor HDO, such as carbon, alumina, TiO₂ and ZrO₂. Acidity is also required for oxygenate activation, including alcohol dehydration.

It is also known that acidity has an effect on the alcohol dehydration, which may favor HDO by dehydration mechanism more hydrogenation, as in alumina-supported catalysts. Further, acidity of some supports can favor the dispersion of the metallic function of HDO.

Preferably, however, the direct mechanism of hydrodeoxygenation is preferred.

Typical pressure conditions range from 5 to 100 bar, preferably 10 to 50 bar, more preferably 20 to 40 bar are sufficient to conversion of the remaining oxygenates (alcohols, phenols, aldehydes, esters) to hydrocarbons being unnecessary higher pressures. Temperatures typical ranges from 200 °C to 400 °C, preferably from 200 °C to 350 °C, more preferably from 250 °C to 325 °C. Lower temperatures lower the hydrodeoxygenation, and higher temperatures can lead to deactivation.

Finally, after the HDO reaction, hydrogenation can be carried out from alkyl aromatic to alkyl naphthenic.

Typical aromatic hydrogenation catalysts can be of Ni or noble metals like Pt, Pd, Ru, Rh, Re. Supports such as alumina, silica-alumina, zeolites, active carbon, Ti, basic oxides, clays.

Typical pressure conditions range from 5 to 100 bar, preferably 10 to 50 bar, more preferably 20 to 40 bar are sufficient to conversion of the remaining oxygenates (alcohols, phenols, aldehydes, esters) to hydrocarbons being unnecessary higher pressures. Temperatures typical ranges from 150 °C to 300 °C, preferably from 150 °C to 250 °C, more preferably from 200 °C to 250 °C. Lower temperatures lower the hydrogenation, and higher temperatures can lead to hydrogenolysis.

As the hydrogenation reaction is exothermic, the recycling of alkyl naphthenics in the reaction load may be necessary, in addition to the use of liquid or gas quench at different points in the reactor.

Further, the HDO and hydrogenation steps of aromatics may be combined in the same reactor. The reactor may have catalysts in separated beds, being the preferred section for the first contact with the load the HDO catalyst. It is also possible to mix HDO and hydrogenation.

Another particular arrangement of the present invention is to carry out the HDO and hydrogenation reactions of aromatics in the same catalyst. Therefore, sites may be combined that have a hydrodeoxygenating function and/or acid function, with hydrogenating function, in preferred configuration of the present invention the support may have the acid dehydration function plus a metal with a hydrogenating function. Any of the sites and components mentioned above can be used for deoxygenation and hydrogenation combined reactions, being known to those skilled in the art. Several options are viable, and variations on possible catalysts for HDO and hydrogenation or combined HDO and hydrogenation steps are provided to processing the coupling reaction effluent. The final product being obtained as a mixture of alkyl aromatics and alkyl naphthenics or just alkyl naphthenics.

Methods for obtaining reagents, methods for separating products, methods of carrying out the reaction in reactors, means of recycling products, means of thermal exchange, are known in the state of the art, the presently described invention not being changed by the use of any other expedients known in the state of the art not listed in the present invention.

It is also clear that the nature of the coupling catalyst, presenting, however, basic and hydrogenating/dehydrogenating sites, is not limitation of the present invention, several heterogeneous or homogeneous catalysts or combinations, are able to catalyze the present invention of ethanol coupling reaction, optionally with CO₂ and methanol, resulting in alkyl aromatic and optionally alkyl naphthenic components, after hydrogenation.

### EXAMPLES:

In the examples below, examples are presented in order to illustrate some particular embodiments of the present invention and should not be interpreted as limiting the same.

### EXAMPLE 1: Testing of catalysts from the state of the art

The catalysts were tested at 350 °C in bench scale, continuous reactor, 10 mL of catalyst bed, after activation for 4 hours in atmosphere of H₂ at 400 °C. The load was ethanol, pressure of 30 bar, LHSV of 1 h⁻¹ and H₂/load ratio of 6 mol H₂/mol ethanol.

Commercial catalyst of CoO (5% by weight) + MoOs (15% by weight) in hydrotalcite (30% by weight of MgO) showed activity for etherification conversion of ethanol to dimethyl ether and hydrogenation of ethylene product from dehydration. Ni catalyst (20% by weight) in MgO showed preferably ethanol hydrogenolysis capacity generating methane.

### EXAMPLE 2: Copper catalysts in basic support

A mass of 100 grams of extruded alumina 1/16 was impregnated with CuCl₂.2H₂O solution in corresponding volume of ethanol to the pore volume of the alumina, in order to obtain 5 grams of Cu. The alumina containing Cu was dried for 24 hours and calcined at 420 °C for 4 hours, obtaining the intermediate Cu/Al.

Several catalysts were obtained by adding to the intermediate basic solutions. Sodium, magnesium, calcium and potassium hydroxides, in the ratios of 5, 10, 20 and 30 g metal/g alumina, the catalysts being subsequently calcined at 420 °C for 4 hours.

Catalysts containing potassium, specifically containing 20 g K/100 g original Al₂O₃ showed higher conversion and selectivity for the Guerbet reaction. Contents greater than 20 g K/100 g Al₂O₃ decreased the mechanical strength of the catalyst. 5% Cu content proved to be sufficient for the dehydrogenation reaction step.

Another copper salt used, nitrate, Cu(NO₃)₂ instead of CuCl₂, the catalyst was significantly less active. Without limiting the invention to a working hypothesis of the reaction, it is believed that Cu(I) is more active for dehydrogenation than Cu(0), obtained mostly from salt nitrate.

### EXAMPLE 3: Tests in a pilot reactor of the ethanol coupling reaction

Alumina supported catalyst was used, which in 100 grams base were added 5 grams of Cu (from CuCl₂) and 20 grams of K (from KOH), prepared as described in EXAMPLE 2.

In a downflow pilot reactor, 100 mL of catalyst from EXAMPLE 3 were added.

The catalyst was activated at a temperature of 400 °C, pressure of 30 bar of H₂ for 4 hours.

The load had different contents of ethanol, 10% ethanol solution in water to 100% ethanol. Temperature test ranged from 360 °C to 460 °C, pressure 30 bar, H2/load ratio from 60 to 420 NL/L of load and LHSV from 0.5 to 6 h⁻¹.

The temperature of 360 °C proved to be insufficient with low conversion of ethanol while at 460 °C the temperature was excessive, increasing the formation of diethyl ether.

The addition of water in the reaction mixture proved to be advantageous, obtaining high conversion of ethanol with very low formation of diethyl ether (DEE). Without water it was only possible to inhibit the formation of DEE with less temperature and high LHSV, however under these conditions the conversion of ethanol it was smaller.

**Table 1: Results of coupling tests**

| **Test** | **% EtOH** | **LHSV, h^{- 1}** | **T, °C** | **H₂/load, NL/L** | **Product % EtOH** | **% DEE** |
|---|---|---|---|---|---|---|
| 19 | 100 | 6 | 400 | 30 | 9.1 | 0.3 |
| 20 | 100 | 4 | 400 | 30 | 74.2 | 0.3 |
| 21 | 100 | 2 | 400 | 60 | 62.4 | 1.0 |
| 18 | 100 | 6 | 430 | 30 | 49.0 | 0.6 |
| 17 | 100 | 4 | 430 | 30 | 48.1 | 0.6 |
| 16 | 100 | 3 | 430 | 30 | 38.5 | 0.9 |
| 15 | 100 | 2 | 430 | 30 | 15.3 | 2.1 |
| 14 | 100 | 1.5 | 430 | 30 | 10.2 | 3.6 |
| 13 | 100 | 1 | 430 | 30 | 5.0 | 7.0 |
| 8 | 100 | 0.5 | 430 | 60 | 0.8 | 22.2 |
| 7 | 100 | 0.5 | 460 | 60 | 2.7 | 23.7 |
| 2 | 100 | 0.5 | 440 | 60 | 0.2 | 21.8 |
| 3 | 100 | 0.5 | 420 | 60 | 2.0 | 15.9 |
| 4 | 100 | 0.5 | 400 | 60 | 9.5 | 10.5 |
| 5 | 100 | 0.5 | 380 | 60 | 46.2 | 4.9 |
| 6 | 100 | 0.5 | 360 | 60 | 65.0 | 2.4 |
| 9 | 50 | 0.5 | 430 | 60 | 0.6 | 2.2 |
| 22 | 10 | 0.5 | 430 | 60 | 0.0 | 0.5 |
| 23 | 10 | 0.5 | 430 | 420 | 0.1 | 0.4 |

Table 2 presents the chromatography results of tests 8, 9, 22 and 23, of conditions described in Table 1. It is observed that adding water from test 8 to 9 decreased the formation of DEE and increased the content of all products. A further increase in water content (test 9 to 22), processing a load with only 10% ethanol resulted in a lower light content, although the benzene content increased, it also decreased C7 and C8 and increased C9 to C17. The effect of the additional H₂ in test 23 was to decrease the total light and increase the content of intermediates C7 to C9, keep high yields of C10 to C15 and decrease C17+.

In general, the content of products C8 to C17 (including aromatics) goes from 47 to 76.25% with the addition of water.

The analysis of light fractions still shows a large amount of olefins and dienes that can be reacted with light aromatics (benzene and toluene) by acid catalysis for alkylation, increasing the yield of fraction C8-C17.

### EXAMPLE 4: Tests in a pilot reactor of the ethanol coupling reaction plus methanol

To evaluate the presence of methanol in the coupling reactions, load tests were carried out containing ethanol and methanol, and ethanol and methanol plus water.

Table 3 presents the results of tests 8, 9, 22, without methanol, and of tests 25 and 27, with methanol in the load.

The results showed that methanol when present in the load reacts mostly with ethanol, forming a significant amount of C3 compounds, alcohols and aldehydes, decreasing the production of aromatic compounds C8+ compared to products without methanol in the load.

The results also show that the presence of water is preferable even with load containing ethanol and methanol.

**Table 2: Results of tests 8, 9, 22 and 23**

| **Tests** | **8** | **9** | **22** | **23** |
|---|---|---|---|---|
| **% EtOH** | 100 | 50 | 10 | 10 |
| **LHSV, h⁻¹** | 0.5 | 0.5 | 0.5 | 0.5 |
| **T, °C** | 430 | 430 | 430 | 430 |
| **H₂/load, NL/L** | 60 | 60 | 60 | 420 |
| **Product % EtOH** | 0.80 | 0.63 | 0.02 | 0.07 |
| **% DEE** | 22.20 | 2.17 | 0.53 | 0.38 |
| **Lights** | 15.10 | 21.32 | 6.18 | 7.09 |
| **Benzene** | 4.30 | 5.78 | 7.88 | 5.18 |
| **C7** | 7.78 | 9.86 | 4.92 | 6.46 |
| **Toluene** | 2.87 | 2.25 | 5.91 | 6.28 |
| **C8** | 5.16 | 7.62 | 4.24 | 6.19 |
| **Ethyl benzene** | 2.93 | 2.91 | 4.26 | 5.31 |
| **Xylenes** | 3.49 | 2.25 | 4.55 | 2.65 |
| **Styrene** | 0.41 | 0.87 | 0.53 | 0.45 |
| **C9** | 7.76 | 10.07 | 11.84 | 13.76 |
| **C10** | 9.33 | 10.58 | 12.67 | 12.13 |
| **C11** | 4.81 | 6.63 | 6.84 | 7.08 |
| **C12** | 3.76 | 4.66 | 6.40 | 6.10 |
| **C13** | 3.44 | 3.96 | 5.49 | 5.08 |
| **C14** | 2.66 | 3.26 | 6.29 | 5.34 |
| **C15** | 1.03 | 1.24 | 2.62 | 2.23 |
| **C16** | 1.52 | 2.26 | 4.80 | 3.77 |
| **C17+** | 0.69 | 1.59 | 5.73 | 4.39 |
| **Sum C8-C17+** | 46.98 | 57.91 | 76.25 | 74.48 |

### EXAMPLE 5: Tests in a pilot reactor of the methanol coupling reaction in final step

Since many C3 compounds were formed in the coupling reaction when methanol was mixed directly with ethanol, the possibility of using methanol after a first ethanol conversion step was evaluated.

Tests were performed using a test product mixture prior to ethanol coupling. The resulting product mixture is presented in Table 4, column load mix 1.

An equal volume of methanol was added to mix 1, resulting in the column load mix 1 + MeOH.

In the same condition of T, LHSV and H₂/load ratio of test 25, the product showed a lower content of C3 compounds from the reaction with EtOH remaining from mix 1 load, with a significant amount of methanol converted to heavier products. The addition of methanol posteriori to a first coupling step showed the generation of smaller levels of C3 compounds in the product compared to the previous example, which shows that it is a preference of the present invention to use methanol in a final step of coupling reaction.

### EXAMPLE 6: Tests with MeOH precursor gas streams

Tests with ethanol plus synthesis gas stream (2 molar H₂:1 molar CO) and with ethanol plus regular CO₂ and H2 (3 molar H₂:1 molar CO₂) showed that the desired reactions occurred similarly to the presence of MeOH in the load.

The example shows that the same CO₂ and H₂ can be used in the coupling reaction.

### EXAMPLE 7: Analysis of gaseous effluents from the ethanol coupling reaction

The analysis of the gaseous effluents from the reactions showed typical levels of 80% H₂, 8 to 14% CO₂, less than 5% CO, typically 1 to 2%, and small methane contents, around 2%, ethane and ethylene, lower than 1%, propane, propylene and other light, totaling 100%. The presence of water reduced the CO content from 2% to less than 0.3%.

The H₂ content was higher in the test with higher H₂ feeding in the load, reaching 94% and causing a decrease mainly in the content of CO₂ to less than 2.5%.

**Table 3: Results of tests 8, 9, 22, 25 and 27**

| **Tests** | **8** | **9** | **22** | **25** | **27** |
|---|---|---|---|---|---|
| **% EtOH** | 100 | 50 | 10 | 50 | 33.333 |
| **% MeOH** | | | | **50** | 33.333 |
| **% H₂O** | | | | 0 | 33.333 |
| **LHSV, h⁻¹** | 0.5 | 0.5 | 0.5 | 0.5 | 0.5 |
| **T, °C** | 430 | 430 | 430 | 430 | 430 |
| **H₂/load, NL/L** | 60 | 60 | 60 | 60 | 60 |
| **Product % EtOH** | 0.80 | 0.63 | 0.02 | 0.80 | 0.86 |
| **Product % MeOH** | 0.00 | 0.00 | 0.00 | 0.00 | 0.00 |
| **% DEE, DME and MeOEt** | 22.20 | 2.17 | 0.53 | 11.52 | 3.27 |
| **Alcohols and aldehydes C3** | - | - | - | 19.27 | 18.41 |
| **Lights** | 15.10 | 21.32 | 6.18 | 8.91 | 11.14 |
| **Benzene** | 4.30 | 5.78 | 7.88 | 1.20 | 1.82 |
| **C7** | 7.78 | 9.86 | 4.92 | 5.44 | 6.56 |
| **Toluene** | 2.87 | 2.25 | 5.91 | 3.68 | 3.49 |
| **C8** | 5.16 | 7.62 | 4.24 | 6.41 | 7.68 |
| **EtBz** | 2.93 | 2.91 | 4.26 | 1.19 | 1.83 |
| **Xylenes** | 3.49 | 2.25 | 4.55 | 5.33 | 4.36 |
| **Styrene** | 0.41 | 0.87 | 0.53 | 0.16 | 0.38 |
| **C9** | 7.76 | 10.07 | 11.84 | 7.86 | 9.03 |
| **C10** | 9.33 | 10.58 | 12.67 | 9.70 | 11.13 |
| **C11** | 4+81 | 6.63 | 6.84 | 7.29 | 8.07 |
| **C12** | 3.76 | 4.66 | 6.40 | 4.25 | 4.53 |
| **C13** | 3.44 | 3.96 | 5.49 | 3.06 | 3.28 |
| **C14** | 2.66 | 3.26 | 6.29 | 1.99 | 2.20 |
| **C15** | 1.03 | 1.24 | 2.62 | 0.75 | 0.70 |
| **C16** | 1.52 | 2.26 | 4.80 | 0.94 | 0.98 |
| **C17+** | 0.69 | 1.59 | 5.73 | 0.40 | 0.36 |
| **Sum C8-C17+: %m** | 46.98 | 57.91 | 76.25 | 49.32 | 54.52 |

**Table 4: Test results of reprocessing load with methanol**

| **Tests** | **Load Mix 1** | **Mix 1 + MeOH** | **28** |
|---|---|---|---|
| **LHSV, h⁻¹** | - | - | 0.5 |
| **T, °C** | - | - | 430 |
| **H₂/load, NL/L** | - | - | 60 |
| **% EtOH (load/product)** | 17.72 | 11.95 | 0.14 |
| **% MeOH** | | 35.87 | 0.30 |
| **% DEE, DME and MeOEt** | 7.14 | 4.42 | 4.18 |
| **Alcohols and aldehydes C3** | - | - | 16.17 |
| **Lights** | 9.40 | 5.39 | 7.61 |
| **Benzene** | 4.12 | 3.35 | 1.87 |
| **C7** | 7.37 | 4.01 | 7.14 |
| **Toluene** | 12.40 | 8.21 | 11.50 |
| **C8** | 5.45 | 3.30 | 5.77 |
| **EtBz** | 1.77 | 1.15 | 2.35 |
| **Xylenes** | 2.86 | 1.92 | 4.48 |
| **Styrene** | 0.52 | 0.34 | 0.11 |
| **C9** | 6.96 | 4.62 | 8.06 |
| **C10** | 8.44 | 5.65 | 9.58 |
| **C11** | 5.02 | 3.00 | 5.35 |
| **C12** | 3.58 | 2.35 | 3.62 |
| **C13** | 2.78 | 1.81 | 3.38 |
| **C14** | 2.26 | 1.33 | 2.35 |
| **C15** | 0.98 | 0.59 | 0.85 |
| **C16** | 1.42 | 0.64 | 1.17 |
| **C17+** | 0.41 | 0.08 | 0.48 |
| **Sum C8-C17+** | 42.43 | 26.78 | 47.54 |

Mass flow totalizers of gaseous and liquid effluents from the reactor point out that typically to each 100 mass units of ethanol fed to the reactor results in 5 masses of H₂, 10 masses of CO₂ and about 1 mass of CO.

### EXAMPLE 8: Tests without H₂ in the reactor feed

Tests without H₂ in the reactor feed showed results similar to tests with H₂.

### EXAMPLE 9: Bench reactor test of the hydrodeoxygenation of the ethanol coupling reaction product

A commercial NiMo catalyst, pre-sulfided, was activated in H₂ atmosphere at 30 bar.

A mixture of conversion tests from the ethanol conversion was used, mixture of products from EXAMPLE 3.

An LHSV of 0.5 h⁻¹, temperature of 320 °C, pressure of 30 bar and H₂/load ratio of 200 mL H₂/mL load. Table 5 shows the test product analysis result. To take into account an expected concentration effect due to the conversion of ethanol to ethene (detected in the gaseous effluent of the unit) and part of the DEE, a comparative load was calculated in the middle column.

There is a decrease in light components and benzene, as well as the elimination of oxygenated compounds, olefins and dienes present in load, and the increase of the corresponding paraffins. Some characterized oxygenated accounted for as light in the table, such as acetaldehyde, acetone, isopropanol, propanol, butanol, butanal, 2-butenal, when hydrodeoxygenation are lost in the depressurization of the product, decreasing the light cut.

Since the catalyst has acidity, there may have been some alkylation of more reactive dienes with benzene to form heavier compounds.

**Table 5: Results of hydrodeoxygenation tests with HDT catalyst**

| **Tests** | **Actual load** | **Load with the same content of EtOH and DEE of the product** | **HDT product** |
|---|---|---|---|
| **LHSV, h⁻¹** | - | - | 0.5 |
| **T, °C** | - | - | 320 |
| **P, bar** | - | - | 30 |
| **H₂/load, NL/L** | - | - | 200 |
| **% EtOH (load/product)** | 16.76 | 0.08 | 0.08 |
| **% DEE** | 7.03 | 3.73 | 3.73 |
| **Lights** | 9.08 | 11.37 | 6.80 |
| **Benzene** | 4.96 | 6.20 | 1.89 |
| **C7** | 6.68 | 8.36 | 7.52 |
| **Toluene** | 11.97 | 14.97 | 16.56 |
| **C8** | 5.52 | 6.90 | 6.14 |
| **EtBz** | 1.74 | 2.17 | 3.07 |
| **Xylenes** | 2.84 | 3.56 | 5.22 |
| **Styrene** | 0.53 | 0.66 | 0.19 |
| **C9** | 7.06 | 8.83 | 9.85 |
| **C10** | 8.52 | 10.66 | 11.98 |
| **C11** | 4.88 | 6.11 | 5.96 |
| **C12** | 3.71 | 4.65 | 4.47 |
| **C13** | 3.07 | 3.84 | 4.55 |
| **C14** | 2.78 | 3.48 | 4.98 |
| **C15** | 0.99 | 1.24 | 2.26 |
| **C16** | 1.57 | 1.96 | 3.20 |
| **C17+** | 0.99 | 1.24 | 2.60 |
| **Sum C8-C17+** | 44.20 | 55.30 | 64.47 |

### EXAMPLE 10: Hydrogenation of alkyl naphthenics

The product obtained in EXAMPLE 9 was used as filler. Test with commercial aromatic hydrogenation catalyst containing 0.4% of Pd and 0.2% Pt supported on alumina shows that at a temperature of 250 °C, LHSV of 1.0 h⁻¹, pressure of 45 bar and 600 NUL of H₂ almost all the aromatic effluents from the sample was hydrogenated to alkyl naphthenics.

It should be noted that, although the present invention has been illustrated by examples of conversion of ethanol to alkyl aromatics and alkyl naphthenics, this may undergo modifications and adaptations by the one skilled in the art, depending on the specific situation, but provided within the inventive scope defined herein.

## Claims

1. PROCESS FOR OBTAINING RENEWABLE AVIATION KEROSENE, **characterized by** comprising the following steps:
(a) feeding to a coupling step an ethanol in water solution plus carbon dioxide load;
(b) coupling step with at least one reactor with coupling catalyst with basic function combined with hydrogenating/dehydrogenating function;
(c) carrying out the coupling step under operating conditions of pressure between 1 to 200 bar, temperature between 150 °C to 550 °C, LHSV between 0.1 and 10 h⁻¹ and H₂/load ratio between 0 and 500 NL/L;
(d) sending the liquid effluent from step (c) to the treatment step;
(e) treatment step where hydrodeoxygenation and hydrogenation reactions occur, recovering product with at least 50% C8+.

2. PROCESS, according to claim 1, **characterized in that** the ethanol comes from a renewable source.

3. PROCESS, according to claim 1, **characterized in that** the carbon dioxide comes from the sugar fermentation.

4. PROCESS, according to claim 1, **characterized in that** the carbon dioxide comes from the recycle of gaseous effluent from the coupling reaction.

5. PROCESS, according to claim 1, **characterized in that** the load further contains methanol, carbon dioxide, synthesis gas or mixtures thereof.

6. PROCESS, according to claim 5, **characterized in that** the methanol is added to the coupling reactor after a first coupling step.

7. PROCESS, according to claim 1, **characterized in that** the ethanol solution has a concentration of 10% ethanol in water up to 100% ethanol.

8. PROCESS, according to claim 1, **characterized in that** the operating conditions of the coupling step are: pressure between 10 to 60 bar, temperature between 250 °C to 450 °C, LHSV between 0.2 to 5 h⁻¹ and H₂/load between 0 to 100 NL/L.

9. PROCESS, according to claim 8, **characterized in that** the operating conditions of the coupling step are: pressure between 20 to 40 bar, temperature between 350 °C to 450 °C, LHSV between 0.5 to 2 h⁻¹ and H₂/load between 20 to 50 NL/L.

10. PROCESS, according to claim 1, **characterized in that** the coupling catalyst with hydrogenating/ dehydrogenating function is selected among transition metals of groups VB, VIB, VIIIB, IB, mainly Cu, Ni, V, Cr, Mo, W, Fe, Ru, Co, Pt and Pd, present in the catalyst in the form of oxides, carbonates, halides, phosphates, carbides, nitrides or even as reduced metals.

11. PROCESS, according to claim 10, **characterized in that** the coupling catalyst with hydrogenating/ dehydrogenating function is copper supported on K-doped Al₂O₃.

12. PROCESS, according to claim 1, **characterized in that** the coupling catalyst with basic function is selected from basic compounds, alkaline and alkaline earth metals of groups IA and IIA in form of oxides, hydroxides, phosphates, carbonates, supported or not on heterogeneous supports such as aluminas, hydrotalcites, zeolites, silicas, silica-aluminas, activated charcoal, mixed oxides, spinels, or basic clays such as limestone, dolomite, magnesite, sepiolite, olivine, anion exchange resins, metal oxides such as zinc oxide and basic organometallics.

13. PROCESS, according to claim 1, **characterized in that** the operating conditions of the hydrodeoxygenation step are: pressure between 10 to 50 bar and temperature between 200 °C to 350 °C.

14. PROCESS, according to claim 13, **characterized in that** the treatment step includes a hydrodeoxygenation with operational conditions of: pressure between 20 to 40 bar and temperature between 250 °C to 325 °C.

15. PROCESS, according to claim 1, **characterized in that** the hydrodeoxygenation catalyst of the treatment step is selected between Mo or W sulfides, promoted by Ni or Co, supported on solids such as alumina, silicas, silica-aluminas, zeolites, hydrotalcites, mixed oxides, spinels, MgO, TiO₂, ZnO, CeO₂, phosphates, sulfonic resins, ZrO₂, sulfated Zr, carbon and active carbon.

16. PROCESS, according to claim 1, **characterized in that** the hydrodeoxygenation catalyst is selected from partially reduced metals as Pt, Pd, Ru, Ni, Cu, Mo, W, Co, Ir, Rh, Au, Ce, Fe, Mn, Ga, Pb and Bi, alone or in mixtures, supported on solids such as alumina, silica, silica-aluminas, zeolites, hydrotalcites, mixed oxides, spinels, MgO, TiO₂, ZnO, CeO₂, phosphates, sulfonic resins, ZrO₂, sulfated Zr, carbon or active carbon.

17. PROCESS, according to claim 16, **characterized in that** the hydrodeoxygenation catalyst is a partially reduced, metal oxide supported on porous solid, such as MoOs, and other metals such as RuO₂, IrO₂, PdO, Rh₂O₃, SnO₂, ZnO, VO₂, TiO₂, CeO₂, CuO, Ag₂O and Au₂O₃.

18. PROCESS, according to claim 16, **characterized in that** the support for the hydrodeoxygenation catalyst is oxophilic, such as alumina, TiO₂ and ZrO₂.

19. PROCESS, according to claim 1, **characterized in that** the treatment step comprises a hydrogenation with a hydrogenation catalyst chosen from Ni or noble metals such as Pt, Pd, Ru, Rh and Re supported on alumina, silica-alumina, zeolites, active carbon, Ti, basic oxides or clays.

20. PROCESS, according to claim 1, **characterized in that** the treatment step comprises a single step of hydrodeoxygenation reaction plus hydrogenation, with a combination of catalysts for the hydrodeoxygenation and hydrogenation reactions in the same catalyst.
